# EUROPEAN PATENT SPECIFICATION

(11) **EP 2 537 474 B1**
(45) Date of publication and mention of the grant of the patent: **14.11.2018**
(21) Application number: 12172940.4
(22) Date of filing: 21.06.2012
(51) Int. Cl.: A61B 17/128

(54) **Surgical clip applier including atraumatic jaw feature**
Chirurgischer Klammerapplikator mit atraumatischer Klemmbackenfunktion
Applicateur d'agrafes chirurgicales avec fonctionnalité de mâchoire atraumatique

(30) Priority: 22.06.2011 US 201161499839 P; 30.04.2012 US 201213459728
(43) Date of publication of application: 26.12.2012
(73) Proprietor: Covidien LP, Mansfield, MA 02048 (US)
(72) Inventor: Creston, Brian, West Haven, CT Connecticut 06516 (US); Zammatoro, Tom, Hamden, CT Connecticut 06518 (US)
(74) Representative: Soames, Candida Jane

(56) References cited:
- DE-U1- 9 413 296
- US-A- 5 474 566
- US-A- 6 139 555
- US-A1- 2004 097 971
- US-A1- 2008 312 670

## Description

### BACKGROUND

### Technical Field

The present disclosure relates to surgical instruments. More particularly, the present disclosure relates to an endoscopic surgical clip applier.

### Description of Related Art

Endoscopic staplers and clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures. Typically, a tube or cannula device is extended into the patient's body through the entrance incision to provide an access port. The port allows the surgeon to insert a number of different surgical instruments therethrough using a trocar and for performing surgical procedures far removed from the incision.

During a majority of these procedures, the surgeon must often terminate the flow of blood or another fluid through one or more vessels. The surgeon will often apply a surgical clip to a blood vessel or another duct to prevent the flow of body fluids therethrough during the procedure. An endoscopic clip applier is known in the art for applying a single clip or a series of clips during a surgical procedure. Such clips are typically fabricated from a biocompatible material and are usually compressed over a vessel. Once applied to the vessel, the compressed clip terminates the flow of fluid therethrough.

Endoscopic clip appliers that are able to apply multiple clips in endoscopic or laparoscopic procedures during a single entry into the body cavity are described in commonly-assigned U.S. Pat. Nos. 5,084,057 and 5,100,420 to Green et al. Another multiple endoscopic clip applier is disclosed in commonly-assigned U.S. Pat. No. 5,607,436 by Pratt et al. These devices are typically, though not necessarily, used during a single surgical procedure. U.S. Pat. No. 5,695,502 to Pier et al., a resterilizable surgical clip applier. The clip applier advances and forms multiple clips during a single insertion into the body cavity. This resterilizable clip applier is configured to receive and cooperate with an interchangeable clip magazine so as to advance and form multiple clips during a single entry into a body cavity.

US 5 474 566 A discloses an apparatus for applying surgical clips including a first jaw including an inner surface oriented defining a channel having a depth that is less than the width of the leg of the surgical clip to be received therein and similar second jaw wherein, when in the closed, approximated position, the legs of the surgical clip come into contact with one another and the inner surface of the first jaw and the inner surface of the second jaw are spaced-apart from one another defining an atraumatic area between the inner surface of the first jaw and the inner surface of the second jaw.

However, during use, the jaws of the surgical clip applier may be urged into contact with one another thus pinching, or trapping tissue therebetween. This is particularly likely in instances where the jaws are actuated in the absence of a surgical clip disposed therebetween, e.g., due to malfunction of the surgical clip applier or lack of clips remaining in the clip magazine. Such an occurrence may result in the cutting or damaging of tissue. It is therefore desirable to provide a surgical clip applier that includes an atraumatic feature configured inhibit tissue from being cut or otherwise damaged by the approximation of the jaws.

### SUMMARY

The invention is defined in claim 1. Preferred embodiments of the invention are defined in the dependent claims. In accordance with one embodiment of the present disclosure, an improved jaw blade for an apparatus for applying surgical clips is provided. The apparatus includes a handle portion, a body portion extending from the handle portion and a jaw blade extending from the body portion at an end opposite the handle portion. Each surgical clip configured for use with the apparatus includes a pair of legs extending parallel to one another. Each leg defines a width as measured in a direction extending orthogonal to a longitudinal axis of the leg and extending orthogonally toward the opposed leg of the surgical clip. The improved jaw blade includes a first leg and a second leg. A first jaw is integrally connected to the first leg and includes an inner surface oriented toward the second leg. The inner surface of the first jaw defines a channel oriented substantially along a longitudinal axis of the first jaw that has a depth that is less than the width of the leg of the surgical clip to be received therein. The second jaw is integrally connected to the second leg and similarly includes an inner surface oriented toward the first leg that defines a channel oriented substantially along a longitudinal axis of the second jaw. The channel of the second jaw has a depth that is less than the width of the leg of the surgical clip to be received therein. The first leg and the second leg each define longitudinal axes that are substantially parallel to one another and a plane therebetween. The first leg and the second leg of the jaw blade are movable in the plane between an open, spaced-apart position for receiving a surgical clip in the channels of the first jaw and the second jaw, and a closed, approximated position for fully forming the surgical clip received within the channels of the first jaw and the second jaw. When the first leg and the second leg of the jaw blade are in the closed, approximated position, the legs of the surgical clip come into contact with one another and the inner surface of the first jaw and the inner surface of the second jaw are spaced-apart from one another to define an atraumatic area between the inner surface of the first jaw and the inner surface of the second jaw.

In one embodiment, each of the jaws defines a clip formation region therein. The atraumatic area is defined between the clip formation regions of the jaws.

In another embodiment, in the closed, approximated position, the clip formation regions of the jaws are spaced-apart from one another along lengths of the clip formation regions, while the jaws contact one another in at least one position along lengths of the jaws outside of the clip formation regions.

In another embodiment, in accordance with the present invention, one or both of the jaws includes a stop disposed proximally of the clip formation regions of the jaws and extending from the inner surface of the jaw toward the other jaw. The stop is configured to contact the other jaw when the jaws are in the closed, approximated position.

In yet another embodiment, one or both of the jaws includes a stop disposed distally of the clip formation regions of the jaws and extending from the inner surface of the jaw toward the other jaw. The stop is configured to contact the other jaw when the jaws are in the closed, approximated position.

In still another embodiment, one or both of the jaws includes a first stop disposed proximally of the clip formation regions of the jaws and one or both of the jaws includes a second stop disposed distally of the clip formation regions of the jaws. The first and second stops extend toward the other jaw member and are configured to contact the other jaw member when the jaws are in the closed, approximated position such that the clip formation regions of the jaws extend between the first and second stops.

Another embodiment of an improved jaw blade for an apparatus for applying surgical clips is also provided in accordance with the present disclosure. The surgical clip apparatus may be configured similarly as described above with respect to the previous embodiments. The improved jaw blade includes a first leg and a second leg. A first jaw is integrally connected to the first leg and a second jaw is integrally connected to the second leg. Each of the first and second jaws includes an inner surface oriented toward the other leg that defines a channel oriented substantially along a longitudinal axis of the jaw. The first leg and the second leg each define longitudinal axes that are substantially parallel to one another and a plane therebetween. The first leg and the second leg of the jaw blade are movable in the plane between an open, spaced-apart position for receiving a surgical clip in the channels of the first jaw and the second jaw, and a closed, approximated position for fully forming the surgical clip received within the channels of the first jaw and the second jaw. When the first leg and the second leg of the jaw blade are in the closed, approximated position, the inner surface of the first jaw and the inner surface of the second jaw are spaced-apart from one another defining an atraumatic area therebetween.

In embodiments, the improved jaw blade may be configured similarly as described above with respect to the previous embodiments.

### BRIEF DESCRIPTION OF THE DRAWINGS

Various embodiments of the subject instrument are described herein with reference to the drawings wherein:
FIG. 1 is a perspective view of a surgical clip applier;
FIG. 2 is a further perspective view of the surgical clip applier of FIG. 1, illustrating a rotation of a shaft thereof;
FIG. 3 is an enlarged, top, perspective view of the jaw assembly of the surgical clip applier of FIGS. 1-2;
FIG. 4 is a plan view of a surgical clip for use with the surgical clip applier of FIGS. 1-2;
FIG. 5A is a top, plan view of jaw members of a prior art jaw assembly shown in a closed, approximated position;
FIG. 5B is a transverse, cross-sectional view of the prior art jaw assembly, as taken through 5B-5B of FIG. 5A, illustrating the formation of a surgical clip about tissue;
FIG. 6 is a side view of another embodiment of a jaw assembly provided in accordance with the present invention, the jaw assembly disposed in a closed, approximated position;
FIG. 7 is a side view of still another embodiment of a jaw assembly provided in accordance with the present invention, the jaw assembly disposed in a closed approximated position;
FIG. 8 is a side view of yet another embodiment of a jaw assembly provided in accordance with the present disclosure, the jaw assembly disposed in a closed, approximated position; and
FIG. 9 is a transverse, cross-sectional view of any of the jaw assemblies of FIGS. 6-9, as taken exemplarily through 9-9 of FIG. 6, illustrating the formation of a surgical clip about tissue.

### DETAILED DESCRIPTION

Embodiments of a surgical clip applier in accordance with the present disclosure will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

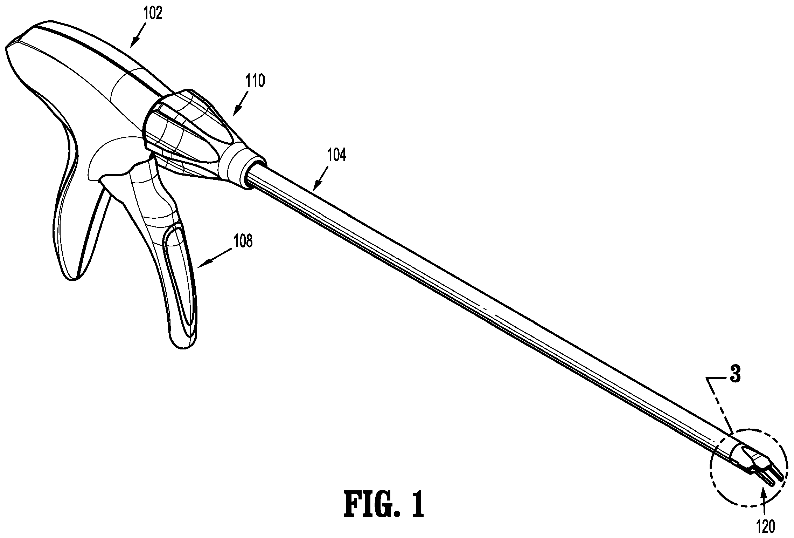

Referring to FIGS. 1-3, a surgical clip applier in accordance with the present disclosure is generally designated as 100. Surgical clip applier 100 generally includes a handle assembly 102 and an endoscopic body portion including a shaft assembly 104 extending distally from handle assembly 102 and having a jaw assembly, or jaw blade 120 disposed at a distal end thereof. A stack of surgical clips (not explicitly shown) is typically loaded and/or retained within shaft assembly 104 in a manner so as to slide therewithin and/or therealong. A complete description of the inner-workings and operation of surgical clip applier 100 can be found in commonly-assigned U.S. Patent Application Serial No. 12/055,446 to Whitfield et. al.

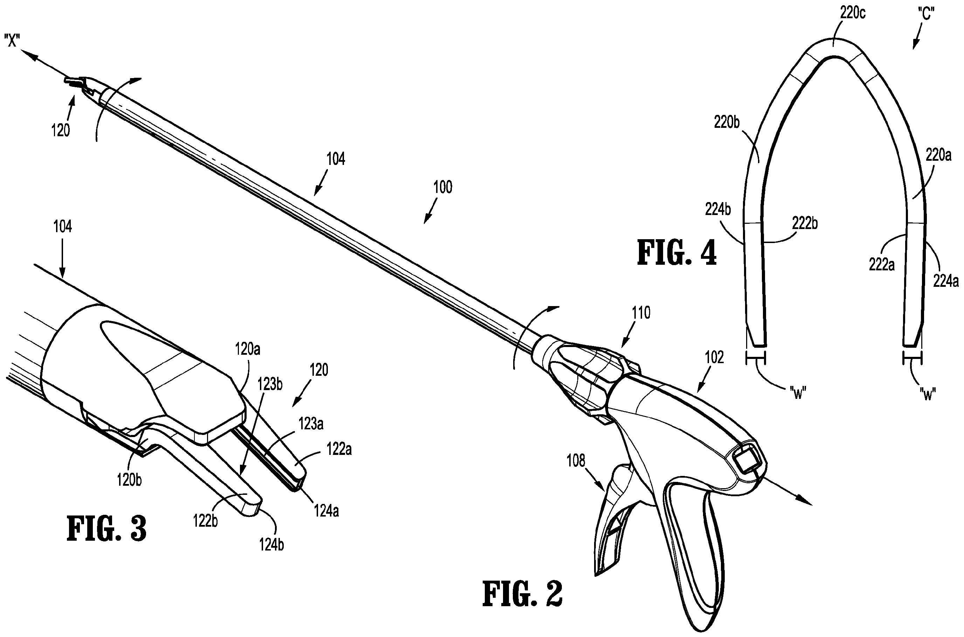

With continued reference to FIGS. 1-3, jaw assembly 120 is mounted in the distal end of shaft assembly 104 and includes a pair of legs 120a, 120b extending distally therefrom. Each leg 120a, 120b includes a jaw 122a, 122b formed integrally therewith and extending distally therefrom. A knob 110 may be rotatably mounted on a distal end of handle assembly 102 and affixed to shaft assembly 104 to transmit and/or provide 360° rotation to shaft assembly 104 and, thus, jaws 122a, 122b about a longitudinal axis "X-X" thereof. As will be described in greater detail hereinbelow, jaws 122a and 122b of jaw assembly 120 are each configured to guide passage of a surgical clip "C" (FIG. 4) therethrough and are moveable relative to one another from an open, spaced-apart position to a closed, approximated position to crimp, or form the surgical clip "C" (FIG. 4) about tissue.

Turning now to FIGS. 3-4, jaws 122a, 122b of jaw assembly 120 each define a respective clip track, or channel 124a, 124b within an inner, or opposed surface 123a, 123b, respectively, thereof. Clip channels 124a, 124b are configured to receive at least a portion of a surgical clip "C" therethrough as jaws 122a, 122b are moved to the closed, approximated position to form the surgical clip "C" about tissue disposed between jaws 122a, 122b. Jaws 122a, 122b are disposed in substantially parallel orientation relative to one another such that clip channels 124a, 124b are substantially parallel relative to one another. Further, jaws 122a, 122b are configured to maintain this substantially parallel orientation throughout movement of jaws 122a, 122b between the open and closed positions, i.e., jaws 122a, 122b are moveable substantially within a single plane between the open and closed positions. Various embodiments of jaws similar to jaws 122a, 122b and configured for use with surgical clip applier 100 (FIGS. 1-2) will be described in greater detail hereinbelow. Further, although the embodiment detailed herein are described and show separately, it is envisioned that the features of any of the embodiments be combinable with one another, to the extent that they are consistent with one another.

Referring now to FIG. 4, a surgical clip "C" configured for use with surgical clip applier 100 (FIG. 1) is shown. Clip "C" includes a pair of substantially parallel, spaced-apart legs 220a, 220b interconnected by a crown, crossbar, or backspan 220c. Each leg 220a, 220b of surgical clip "C" defines a width "w" as measured transversely, or orthogonally across each of the legs 220a, 220b, in a plane defined by legs 220a, 220b. An inwardly facing surface 222a, 222b of each leg 220a and 220b, respectively, defines a generally flat geometry such that upon forming of clip "C" about a body tissue or vessel, inwardly facing surfaces 222a and 222b of legs 220a and 220b, respectively, consistently and evenly clamp around the body tissue or vessel.

Outwardly facing surfaces 224a and 224b of legs 220a and 220b, respectively, may define a generally circular transverse cross-sectional configuration, or any other suitable configuration, and are configured to be at least partially received within clip channels 124a, 124b (FIG. 3) of jaws 122a, 122b (FIG. 3), respectively. In particular, outwardly-facing surfaces 224a and 224b may be shaped complementarily to clip channels 124a, 124b (FIG. 3) of jaws 122a, 122b (FIG. 3), respectively, to facilitate translation of clip "C" through clip channels 124a, 124b (FIG. 3) as jaws 122a, 122b (FIG. 3) are moved to the approximated position to form clip "C" about tissue. Backspan 220c may be configured similarly to legs 220a, 220b to form a substantially consistent configuration along the entire outwardly facing surface of clip "C," although other configurations are contemplated. A more detailed discussion of complementary-shaped clips and clip channels that may be used in accordance with the present disclosure can be found in commonly-owned U.S. Provisional Patent Appl. No. 61/409,131 filed on November 2, 2010.

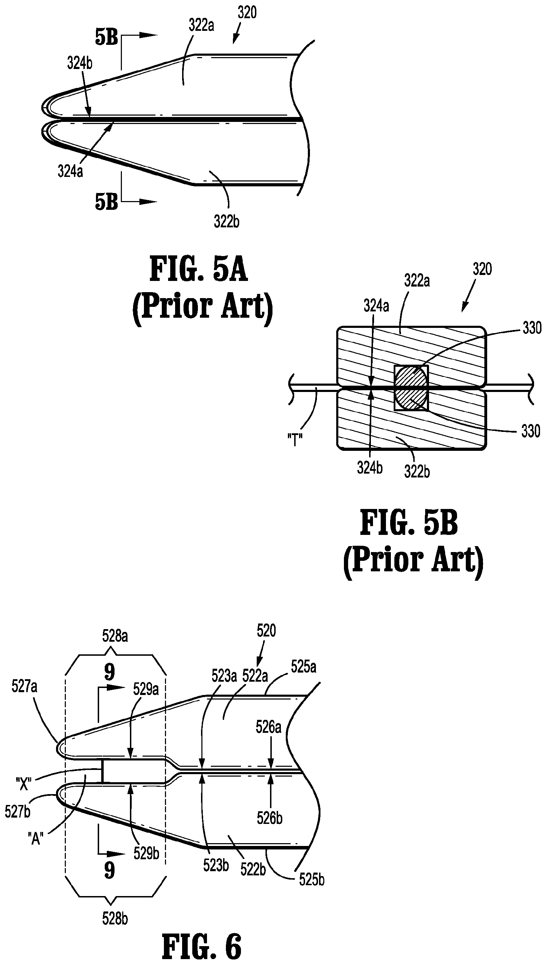

Turning now to FIGS. 5A-5B, a prior art jaw assembly 320 is shown. Jaw assembly 320 includes a pair of jaws 322a, 322b moveable between an open, spaced-apart position and a closed, approximated position to form a surgical clip 330 about tissue "T." As shown in FIG. 5A, in the closed, approximated position, jaws 322a, 322b are fully approximated, or closed. In other words, in the closed, approximated position, opposed surfaces 324a, 324b of jaws 322a, 322b, respectively, of jaw assembly 320 contact one another substantially along the lengths thereof. As a result of this configuration, when jaws 322a, 322b are approximated about tissue "T" to form surgical clip 330 about tissue "T," the inner or opposed surfaces 324a, 324b of jaws 322a, 322b, respectively, are urged toward this fully closed position such that tissue "T" is squeezed, or pinched therebetween, which may sever, lacerate, or otherwise damage tissue "T."

Referring now to FIG. 6, an embodiment of a jaw assembly 520 for use with surgical clip applier 100 (FIGS. 1-2), according to an embodiment of the present disclosure, is shown. Jaw assembly 520 is similar to jaw assembly 120 (FIGS. 1-3) discussed above and thus will only be described in detail herein to the extent necessary to identify differences in construction and/or operation thereof. As seen in FIG. 6, jaw assembly 520 includes first and second jaws 522a, 522b, respectively. First and second jaws 522a, 522b, respectively, are disposed in substantially parallel orientation relative to one another and are moveable relative to one another between an open, spaced-apart position and a closed, approximated position for forming a surgical clip "C" (FIG. 4) about tissue.

As shown in FIG. 6, each jaw 522a, 522b defines an inner, opposed surface 523a, 523b, respectively, and includes a proximal portion 525a, 525b and a distal end 527a, 527b, respectively. A clip formation region 528a, 528b is defined between the proximal portions 525a, 525b and distal ends 527a 527b, respectively, of each jaw 522a, 522b. The surgical clip "C" (FIG. 4) is fully formed about tissue in these clip formation regions 528a, 528b. More particularly, as jaws 522a, 522b are moved toward the closed, approximated position, legs 220a, 220b of surgical clip "C" are held in the clip channels (not explicitly shown) of jaws 522a, 522b, within clip formation regions 528a, 528b, and are moved toward one another. Ultimately, upon reaching the approximated, closed position of jaws 522a, 522b, inwardly facing surfaces 222a, 222b of legs 220a and 220b, respectively, of surgical clip "C" are contacting one another or are in relative close proximity to one another, substantially along the lengths thereof to clamp tissue "T" therebetween (see FIG. 4). As can be appreciated, during formation of surgical clip "C" about tissue, tissue is likewise disposed between clip formation regions 528a, 528b of jaws 522a, 522b, respectively, such that surgical clip "C" may be formed thereabout.

Continuing with reference to FIG. 6, as mentioned above, jaws 522a, 522b each include a respective proximal portion 525a, 525b, distal end 527a, 527b, and clip formation region 528a, 528b defined therebetween. A cut-out, or recess is defined within inner, opposed surfaces 523a, 523b of jaws 522a, 522b, respectively, along the respective clip formation regions 528a, 528b thereof to define an atraumatic area "A" between inner, opposed surfaces 523a, 523b of jaws 522a, 522b, respectively and longitudinally along the clip formation regions 528a, 528b, respectively, thereof. Alternatively, the cut-out, or recess may be formed within only one of the jaws 522a, 522b, or additional material may be formed, or disposed on inner, opposed surfaces 523a, 523b of one or both of jaws 522a, 522b, respectively, outside of the clip formation regions 528a, 528b, respectively, to achieve the same purpose.

As can be appreciated due to the above-described configuration of jaws 522a, 522b, when jaws 522a, 522b are moved to the closed, approximated position, as shown in FIG. 6, to form surgical clip "C" (FIG. 4) about tissue, the segments 529a, 529b of inner, opposed surfaces 523a, 523b of jaws 522a, 522b, respectively, extending along respective clip formation regions 528a, 528b thereof, are spaced a transverse distance apart from one another, thus defining the atraumatic area "A" therebetween (due to the recess, or cut-outs defined therein). On the other hand, the segments 526a, 526b of inner, opposed surfaces 523a, 523b of jaws 522a, 522b, respectively, extending proximally from clip formation regions 528a, 528b, respectively, i.e., extending along proximal portions 525a, 525b of jaws 522a, 522b, respectively, thereof are moved into contact with one another when jaws 522a, 522b are moved to the closed, approximated position (since these segments do not include recesses, or cut-outs defined therein).

In use, jaws 522a, 522b are positioned about tissue such that tissue is disposed between the clip formation regions 528a, 528b of jaws 522a, 522b, respectively. Thereafter, jaws 522a, 522b are moved to the closed, approximated position following distal advancement of surgical clip "C" through jaws 522a, 522b such that surgical clip "C" is disposed within clip formation regions 528a, 528b of jaws 522a, 522b, respectively, and such that inwardly facing surfaces 222a, 222b of legs 220a and 220b, respectively, of surgical clip "C" are contacting one another or in relative close proximity to one another substantially along the lengths thereof to clamp tissue "T" therebetween (see FIG. 4). When jaws 522a, 522b are moved to the closed, approximated position to form surgical clip "C" (FIG. 4) about tissue, the segments 529a, 529b of inner, opposed surfaces 523a, 523b of jaws 522a, 522b, respectively, extending along respective clip formation regions 528a, 528b thereof, are spaced-apart from one another a distance "X," thus defining the atraumatic area "A" therebetween. The atraumatic area "A," defined between clip formation regions 528a, 528b of jaws 522a, 522b, respectively, inhibits the pinching of tissue between opposed surfaces 523a, 523b of jaws 522a, 522b and, thus, reduces the likelihood that tissue will be damaged during formation of surgical clip "C" (FIG. 4) thereabout.

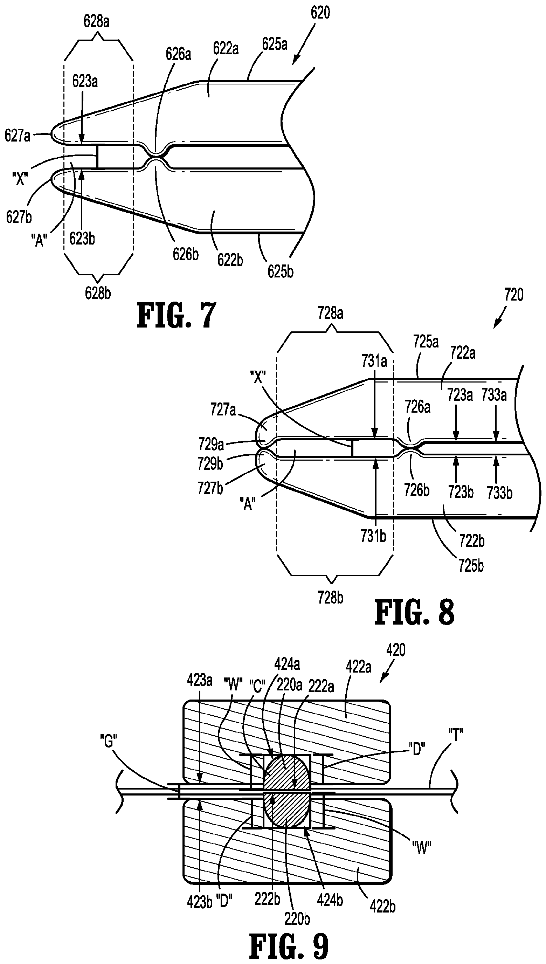

Turning now to FIG. 7, another embodiment of a jaw assembly 620 configured for use with surgical clip applier 100 (FIGS. 1-2) is shown, in accordance with the present invention. Jaw assembly 620 is similar to jaw assembly 120 (FIGS. 1-3) discussed above and thus will only be described in detail herein to the extent necessary to identify differences in construction and/or operation thereof. Jaw assembly 620 includes first and second jaws 622a, 622b, respectively, that are disposed in substantially parallel orientation relative to one another and are moveable relative to one another between an open, spaced-apart position and a closed, approximated position for forming a surgical clip "C" (FIG. 4) about tissue.

As shown in FIG. 7, each jaw 622a, 622b defines an inner, opposed surface 623a, 623b, respectively, and includes a proximal portion 625a, 625b and a distal end 627a, 627b, respectively. A clip formation region 628a, 628b is defined between the proximal portions 625a, 625b and distal ends 627a 627b, respectively, of each jaws 622a, 622b. Similarly as described above with respect to jaw assembly 520 (FIG. 6), the surgical clip "C" (FIG. 4) is fully formed about tissue between the clip formation regions 628a, 628b of jaws 622a, 622b, respectively. Further, also as mentioned above, tissue is positioned between clip formation regions 628a, 628b of jaws 622a, 622b, respectively, such that surgical clip "C" may be formed thereabout.

Continuing with reference to FIG. 7, jaws 622a, 622b each include a stop, or protrusion 626a, 626b disposed on proximal portions 625a, 625b of jaws 622a, 622b, respectively, and extending from inner, opposed surfaces 623a, 623b thereof toward the other jaw 622a, 622b. In other words, protrusions 626a, 626b are positioned proximally of clip formation regions 628a, 628b of jaws 622a, 622b, respectively. Protrusions 626a, 626b are aligned with one another and are configured to contact one another upon moving jaws 622a, 622b to the closed, approximated position, as will be described in greater detail below. However, it is also envisioned that only one of jaws 622a, 622b needs to include a protrusion 626a, 626b extending therefrom or that protrusions 626a, 626b be offset relative to one another to achieve the same purpose.

As shown in FIG. 7, when jaws 622a, 622b are moved to the closed, approximated position to form surgical clip "C" (FIG. 4) about tissue, protrusions 626a, 626b contact one another to inhibit further approximation of jaws 622a, 622b. As such, in this closed, approximated position, opposed surfaces 623a, 623b, respectively, extending along respective clip formation regions 628a, 628b thereof, are spaced-apart from one another a distance "X," thus defining the atraumatic area "A" therebetween. Similarly as discussed above, this atraumatic area "A" defined between clip formation regions 628a, 628b of jaws 622a, 622b, respectively, inhibits the pinching of tissue between opposed surfaces 623a, 623b of jaws 622a, 622b and, thus, reduces the likelihood that tissue will be damaged during formation of surgical clip "C" (FIG. 4) thereabout.

Turning now to FIG. 8, another embodiment of a jaw assembly 720 configured for use with surgical clip applier 100 (FIGS. 1-2) is shown, in accordance with the present invention. Jaw assembly 720 is similar to jaw assemblies 120 (FIGS. 1-3), 520 (FIG. 7), and 620 (FIG. 8), discussed above and thus will only be described in detail herein to the extent necessary to identify differences in construction and/or operation thereof. More specifically, jaw assembly 720 includes features of both jaw assembly 520 (FIG. 7) and jaw assembly 620 (FIG. 8), as will be described in greater detail below, including first and second jaws 722a, 722b, respectively, that are disposed in substantially parallel orientation relative to one another and are moveable relative to one another between an open, spaced-apart position and a closed, approximated position for forming a surgical clip "C" (FIG. 4) about tissue.

As shown in FIG. 8, each jaw 722a, 722b defines an inner, opposed surface 723a, 723b, respectively, and includes a proximal portion 725a, 725b, a distal end 727a, 727b, and a clip formation region 728a, 728b defined between the proximal portions 725a, 725b and distal ends 727a 727b, respectively, of each jaw 722a, 722b. The surgical clip "C" (FIG. 4) is fully formed about tissue in these clip formation regions 728a, 728b, as discussed in detail above with respect to previous embodiments.

Continuing with reference to FIG. 8, a cut-out, or recess is defined within inner, opposed surfaces 723a, 723b of jaws 722a, 722b, respectively, along the respective clip formation regions 728a, 728b thereof to define an atraumatic area "A" between inner, opposed surfaces 723a, 723b of jaws 722a, 722b, respectively and longitudinally along the clip formation regions 728a, 728b, respectively, thereof.

Jaws 722a, 722b each further include a first stop, or protrusion 726a, 726b disposed on proximal portions 725a, 725b thereof and extending from inner, opposed surfaces 723a, 723b thereof toward the other jaw 722a, 722b. Jaws 722a, 722b each further include a second stop, or protrusion 729a, 729b similarly configured and disposed distally of clip formation regions 728a, 728b at distal ends 727a, 727b, respectively, thereof. Accordingly, first and second sets of protrusions 726, 726b and 729a, 729b, respectively, surround clip formation regions 728a, 728b on either longitudinal end thereof. The first protrusions 726a, 726b are aligned with one another and the second protrusions 729a, 729b are aligned with one another such that first protrusions 726a, 726b contact one another and second protrusions 729a, 729b contact one another upon moving jaws 722a, 722b to the closed, approximated position.

In use, when jaws 722a, 722b are moved to the closed, approximated position, as shown in FIG. 8, to form surgical clip "C" (FIG. 4) about tissue, the segments 731a, 731b of inner, opposed surfaces 723a, 723b of jaws 722a, 722b, respectively, extending along respective clip formation regions 728a, 728b thereof, are spaced-apart from one another a distance "X," thus defining an atraumatic area "A" therebetween.

Atraumatic area "A," defining gap distance "X1" between clip formation regions 728a, 728b of jaws 722a, 722b, respectively, is formed partly due to the recesses, or cut-outs defined within jaws 722a, 722b, as described above, and partly due to the contact between first set of protrusions 726a, 726b and the contact between second set of protrusions 729a, 729b when jaws 722a, 722b are approximated, which inhibits further approximation of jaws 722a, 722b. Similarly as above with respect to the previous embodiments, atraumatic area "A" inhibits the pinching of tissue between opposed surfaces 723a, 723b of jaws 722a, 722b and, thus, reduces the likelihood that tissue will be damaged during formation of surgical clip "C" (FIG. 4) thereabout.

With reference now to FIG. 9, which is a transverse cross-section of jaw assembly 520 as taken through 9-9 of FIG. 6 (and which applies equally to jaw assemblies 620 and 720 described above), jaws 522a, 522b are shown in the approximated, closed position forming surgical clip "C" about tissue "T." As seen in FIG. 9, clip channels 524a, 524b each define a depth "D" that is less than a width "w" of legs 220a, 220b of surgical clip "C" such that, when surgical clip "C" is disposed within clip channels 524a, 524b of jaws 522a, 522b, respectively, legs 220a, 220b of surgical clip "C" extend or project from inner, opposed surfaces 523a, 523b of jaws 522a, 522b, respectively, toward the other leg 220a, 220b of surgical clip "C."

Accordingly, as jaws 522a, 522b are moved from the open, spaced-apart position to the closed, approximated position, inner surfaces 222a, 222b of legs 220a, 220b of surgical clip "C" are brought into approximation with one another. More particularly, upon movement of jaws 522a, 522b to the closed, approximated position, inwardly facing surfaces 222a, 222b of legs 220a and 220b, respectively, of surgical clip "C" are moved into abutment or approximate abutment with one another such that the inner surfaces 222a, 222b thereof contact or are in relative close proximity to one another substantially along the lengths thereof to consistently and evenly clamp tissue "T" therebetween.

As can be appreciated, due to the dimensions of clip channels 524a, 524b relative to legs 220a, 220b, respectively, of surgical clip "C," i.e., due to the fact that the width "w" of each of legs 220a, 220b is greater than the depth "D" of the respective clip channels 524a, 524b, when legs 220a and 220b of surgical clip "C" are moved to the abutting, contacting, approximate abutting, or relatively close position, to clamp tissue therebetween, jaws 522a, 522b of jaw assembly 520 remain spaced-apart from one another to define an atraumatic area "A," therebetween defining a gap distance "X." In other words, since legs 220a, 220b of surgical clip "C" extend or project inwardly from each of jaws 522a, 522b toward one another, jaws 522a, 522b need not be fully closed, i.e., need not be brought into contact with one another substantially along the lengths thereof, to fully form surgical clip "C" about tissue "T." This atraumatic area "A," defined between jaws 522a, 522b inhibits or reduces the incidents of pinching of tissue "T" between jaws 522a, 522b and, thus, reduces the likelihood that tissue "T" will be damaged during formation of surgical clip "C" thereabout.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure.

## Claims

1. An apparatus (100) for applying surgical clips, the apparatus including a handle portion (102), a body portion (104) extending from the handle portion, a jaw blade (120) extending from the body portion at an end opposite the handle portion, the jaw blade comprising:
a first leg and a second leg;
a first jaw (622a, 722a) integrally connected to the first leg, the first jaw including an inner surface (623a, 723a) oriented toward the second leg, a proximal portion (625a, 725a), a distal end (627a, 727a) and a clip formation region 628a, 728a) defined between the proximal portion and the distal end, the inner surface of the first jaw defining a channel oriented substantially along a longitudinal axis of the first jaw; and
a second jaw (622b, 722b) integrally connected to the second leg, the second jaw including an inner surface (623b, 723b) oriented toward the first leg, a proximal portion (625b, 725b), a distal end (627b, 727b) and a clip formation region (628b, 728b) defined between the proximal portion and the distal end, the inner surface of the second jaw defining a channel oriented substantially along a longitudinal axis of the second jaw;
wherein the first leg and the second leg each define a longitudinal axis being substantially parallel to one another and a plane therebetween, the first leg and the second leg of the jaw blade being movable in the plane between an open, spaced-apart position for receiving a surgical clip (C) in the channels of the first jaw and the second jaw, and a closed, approximated position for fully forming the surgical clip received within the channels of the first jaw and the second jaw; and
wherein, when the first leg and the second leg of the jaw blade are in the closed, approximated position, the inner surface of the first jaw and the inner surface of the second jaw are spaced-apart from one another defining an atraumatic area therebetween; **characterized in that**:
at least one of the jaws (622a, 722a) includes a stop (626a, 626b, 726a, 726b) disposed proximally of the clip formation region (628a, 628b, 728a, 728b) of the jaw and extending from the inner surface of the at least one jaw, the stop being configured to contact the other jaw when the jaws are in the closed, approximated position whereby in the closed, approximated position, the clip formation regions of the jaws are spaced-apart from one another along lengths of the clip formation regions, and, wherein, in the closed, approximated position, the jaws contact one another in at least one position along lengths of the jaws outside of the clip formation regions.

2. The apparatus of claim 1, wherein at least one of the jaws includes a stop (729a, 729b) disposed distally of the clip formation regions of the jaws and extending from the inner surface of the at least one jaw toward the other jaw, the stop configured to contact the other jaw when the jaws are in the closed, approximated position.

3. The apparatus of claim 1 wherein at least one of the jaws includes the first stop (726a, 726b) disposed proximally of the clip formation regions of the jaws and wherein at least one of the jaws includes a second stop (729a, 729b) disposed distally of the clip formation regions of the jaws, the first and second stops extending toward the other jaw member and configured to contact the other jaw member when the jaws are in the closed, approximated position such that the clip formation regions of the jaws extend between the first and second stops.

4. The apparatus of any one of the preceding claims, wherein the channel of the first jaw has a depth that is less than the width of the leg of the surgical clip to be received therein; and
the channel of the second jaw has a depth that is less than the width of the leg of the surgical clip to be received therein.

## Patentansprüche

1. Vorrichtung (100) zur Anbringung chirurgischer Klammern, wobei die Vorrichtung einen Handgriffabschnitt (102), einen sich von dem Handgriffabschnitt erstreckenden Körperabschnitt (104), eine sich von dem Körperabschnitt an einem dem Handgriffabschnitt gegenüberliegenden Ende erstreckende Klemmbackenklinge (120) aufweist, wobei die Klammbackenklinge umfasst:
ein erstes Bein und ein zweites Bein;
eine erste Klemmbacke (622a, 722a), die mit dem ersten Bein integral verbunden ist, wobei die erste Klemmbacke eine zu dem zweiten Bein hin ausgerichtete Innenfläche (623a, 723a), einen proximalen Abschnitt (625a, 725a), ein distales Ende (627a, 727a) und einen zwischen dem proximalen Abschnitt und dem distalen Ende definierten Klammerformungsbereich (628a, 728a) aufweist, wobei die Innenfläche der ersten Klemmbacke einen Kanal definiert, der im Wesentlichen entlang einer Längsachse der ersten Klemmbacke ausgerichtet ist; und
eine zweite Klemmbacke (622b, 722b), die mit dem zweiten Bein integral verbunden ist, wobei die zweite Klemmbacke eine zu dem ersten Bein hin ausgerichtete Innenfläche (623b, 723b), einen proximalen Abschnitt (625b, 725b), ein distales Ende (627b, 727b) und einen zwischen dem proximalen Abschnitt und dem distalen Ende definierten Klammerformungsbereich (628b, 728b) aufweist, wobei die Innenfläche der zweiten Klemmbacke einen Kanal definiert, der im Wesentlichen entlang einer Längsachse der zweiten Klammbacke ausgerichtet ist;
wobei das erste Bein und das zweite Bein jeweils eine Längsachse, die im Wesentlichen parallel zueinander sind, und eine Ebene dazwischen definieren, wobei das erste Bein und das zweite Bein der Klemmbackenklinge in der Ebene zwischen einer offenen, voneinander beabstandeten Position zur Aufnahme einer chirurgischen Klammer (C) in die Kanäle der ersten Klemmbacke und der zweiten Klemmbacke und einer geschlossenen, angenäherten Position zur vollständigen Formung der in den Kanälen der ersten Klemmbacke und der zweiten Klemmbacke aufgenommenen, chirurgischen Klammer beweglich sind; und
wobei, wenn sich das erste Bein und das zweite Bein der Klemmbackenklinge in der geschlossenen, angenäherten Position befinden, die Innenfläche der ersten Klemmbacke und die Innenfläche der zweiten Klemmbacke voneinander beabstandet sind und einen atraumatischen Bereich dazwischen definieren; **dadurch gekennzeichnet, dass**:
zumindest eine der Klemmbacken (622a, 722a) einen Anschlag (626a, 626b, 726a, 726b) aufweist, der proximal von dem Klammerformungsbereich (628a, 628b, 728a, 728b) der Klemmbacke angeordnet ist und sich von der Innenfläche der zumindest einen Klemmbacke erstreckt, wobei der Anschlag konfiguriert ist, um die andere Klemmbacke zu kontaktieren, wenn sich die Klemmbacken in der geschlossenen, angenäherten Position befinden, wobei in der geschlossenen, angenäherten Position die Klammerformungsbereiche der Klemmbacken entlang der Längen der Klammerformungsbereiche voneinander beabstandet sind und wobei sich in der geschlossenen, angenäherten Position die Klemmbacken in zumindest einer Position entlang der Längen der Klemmbacken außerhalb der Klammerformungsbereiche gegenseitig berühren.

2. Vorrichtung nach Anspruch 1, wobei zumindest eine der Klemmbacken einen Anschlag (729a, 729b) aufweist, der distal von den Klammerformungsbereichen der Klemmbacken angeordnet ist und sich von der Innenseite der zumindest einen Klemmbacke zu der anderen Klemmbacke hin erstreckt, der Anschlag konfiguriert, um die andere Klemmbacke zu kontaktieren, wenn sich die Klemmbacken in der geschlossenen, angenäherten Position befinden.

3. Vorrichtung nach Anspruch 1, wobei zumindest eine der Klemmbacken den ersten Anschlag (726a, 726b) aufweist, der proximal von den Klammerformungsbereichen der Klemmbacken angeordnet ist, und wobei zumindest eine der Klemmbacken einen zweiten Anschlag (729a, 729b) aufweist, der distal von den Klammerformungsbereichen der Klemmbacken angeordnet ist, wobei sich die ersten und zweiten Anschläge zu dem anderen Klemmbackenelement hin erstrecken und konfiguriert sind, um das andere Klemmbackenelement zu kontaktieren, wenn sich die Klemmbacken in der geschlossenen, angenäherten Position befinden, so dass sich die Klammerformungsbereiche der Klemmbacken zwischen den ersten und zweiten Anschlägen erstrecken.

4. Vorrichtung nach einem der vorstehenden Ansprüche,
wobei der Kanal der ersten Klemmbacke eine Tiefe hat, die geringer als die Breite des Beins der darin aufzunehmenden chirurgischen Klammer ist; und
der Kanal der zweiten Klemmbacke eine Tiefe hat, die geringer als die Breite des Beins der darin aufzunehmenden chirurgischen Klammer ist.

## Revendications

1. Appareil (100) pour appliquer des agrafes chirurgicales, l'appareil incluant une partie de poignée (102), une partie de corps (104) s'étendant depuis la partie de poignée, une lame de mâchoire (120) s'étendant depuis la partie de corps à une extrémité opposée à la partie de poignée, la lame de mâchoire comprenant :
une première jambe et une seconde jambe ;
une première mâchoire (622a, 722a) raccordée d'un seul tenant à la première jambe, la première mâchoire incluant une surface intérieure (623a, 723a) orientée vers la seconde jambe, une partie proximale (625a, 725a), une extrémité distale (627a, 727a) et une région de formation d'agrafe (628a, 728a) définie entre la partie proximale et l'extrémité distale, la surface intérieure de la première mâchoire définissant un canal orienté sensiblement le long d'un axe longitudinal de la première mâchoire ; et
une seconde mâchoire (622b, 722b) raccordée d'un seul tenant à la seconde jambe, la seconde mâchoire incluant une surface intérieure (623b, 723b) orientée vers la première jambe, une partie proximale (625b, 725b), une extrémité distale (627b, 727b) et une région de formation d'agrafe (628b, 728b) définie entre la partie proximale et l'extrémité distale, la surface intérieure de la seconde mâchoire définissant un canal orienté sensiblement le long d'un axe longitudinal de la seconde mâchoire ;
dans lequel la première jambe et la seconde jambe définissent chacune un axe longitudinal en étant sensiblement parallèles l'une à l'autre et un plan entre elles, la première jambe et la seconde jambe de la lame de mâchoire étant mobiles dans le plan entre une position ouverte, distante pour recevoir une agrafe chirurgicale (C) dans les canaux de la première mâchoire et de la deuxième mâchoire, et une position fermée proche pour former complètement l'agrafe chirurgicale reçue dans les canaux de la première mâchoire et de la seconde mâchoire ; et
dans lequel, lorsque la première jambe et la seconde jambe de la lame de mâchoire sont dans la position fermée proche, la surface intérieure de la première mâchoire et la surface intérieure de la seconde mâchoire sont espacées l'une de l'autre en définissant une zone atraumatique entre elles ; **caractérisé en ce que** :
au moins l'une des mâchoires (622a, 722a) inclut un arrêt (626a, 626b, 726a, 726b) disposé sur le plan proximal de la région de formation d'agrafe (628a, 628b, 728a, 728b) de la mâchoire et s'étendant depuis la surface intérieure de l'au moins une mâchoire, l'arrêt étant configuré pour entrer en contact avec l'autre mâchoire lorsque les mâchoires sont dans la position fermée proche, selon lequel dans la position fermée proche, les régions de formation d'agrafes des mâchoires sont espacées l'une de l'autre le long de longueurs des régions de formation d'agrafes, et, dans lequel, dans la position fermée proche, les mâchoires entrent en contact l'une avec l'autre dans au moins une position le long de longueurs hors des régions de la formation d'agrafe.

2. Appareil selon la revendication 1, dans lequel au moins l'une des mâchoires inclut un arrêt (729a, 729b) disposé sur le plan distal des régions de formation d'agrafe des mâchoires et s'étendant de la surface intérieure de l'au moins une mâchoire vers l'autre mâchoire, l'arrêt étant configuré pour entrer en contact avec l'autre mâchoire lorsque les mâchoires sont dans la position fermée proche.

3. Appareil selon la revendication 1 dans lequel au moins l'une des mâchoires inclut le premier arrêt (726a, 726b) disposé sur le plan proximal des régions de formation d'agrafe des mâchoires et dans lequel au moins l'une des mâchoires inclut un second arrêt (729a, 729b) disposé sur le plan distal des régions de formation d'agrafe des mâchoires, les premier et second arrêts s'étendant vers l'autre élément de mâchoire et étant configurés pour entrer en contact avec l'autre élément de mâchoire lorsque les mâchoires sont dans la position fermée proche de sorte que les régions de formation d'agrafe des mâchoires s'étendent entre les premier et second arrêts.

4. Appareil selon l'une quelconque des revendications précédentes, dans lequel le canal de la première mâchoire a une profondeur qui est inférieure à la largeur de la jambe de l'agrafe chirurgicale à recevoir dans celui-ci ; et
le canal de la seconde mâchoire a une profondeur qui est inférieure à la largeur de la jambe de l'agrafe chirurgicale à recevoir dans celui-ci.
